# EUROPEAN PATENT APPLICATION

(11) **EP 3 438 669 A1**
(43) Date of publication of application: **06.02.2019**
(21) Application number: 17775381.1
(22) Date of filing: 30.03.2017
(51) Int. Cl.: G01N 33/68, C07K 16/42, C12Q 1/06, G01N 33/53

(54) **METHOD FOR DETERMINING SUSCEPTIBILITY TO DIABETES**

(30) Priority: 30.03.2016 JP 2016068695
(71) Applicant: Matsumori, Akira, Minoh-shi, Osaka 562-0005 (JP)
(72) Inventor: Matsumori, Akira, Minoh-shi, Osaka 562-0005 (JP)
(74) Representative: Müller-Boré & Partner Patentanwälte PartG mbB
(86) International application number: PCT/JP2017/013203
(87) International publication number: WO 2017/170847

(57) **Abstract**

It is an object to provide a biomarker for diabetes, which is not affected by a meal or the like, and is hardly metabolized during storage after blood collection. In addition, it is an object to provide a method of detecting the presence or absence of activation of immunoglobulin-producing cells in a diabetic patient. The concentrations of a kappa chain and a lambda chain, which are included in free immunoglobulin light chains, are measured, and when the ratio of the concentration of the kappa chain to the concentration of the lambda chain, or the absolute value of a difference between the concentration of the kappa chain and the concentration of the lambda chain is lower than a threshhold value, it is determined that diabetes is present and immunoglobulin-producing cells are activated.

## Description

### Technical Field

The present invention relates to a method of determining morbidity of diabetes, a method of detecting the presence or absence of activation of immunoglobulin-producing cells for a diabetic patient, and a test reagent and a test kit to be used for those methods.

### Background Art

Hitherto, free immunoglobulin light chains (FLCs), which include a kappa chain and a lambda chain, have been used for diagnosis of blood diseases, such as myeloma and amyloidosis. In myeloma, depending on kinds of cells that undergo tumorigenic transformation, whereas in a tumor producing the immunoglobulin kappa light chain, the kappa chain increases to raise a kappa chain/lambda chain ratio, in a tumor producing the immunoglobulin lambda light chain, the lambda chain increases to reduce the kappa chain/lambda chain ratio.

In addition, also in diseases other than myeloma, it has been reported that the FLCs serve as a useful marker. For example, in Patent Literature 1, there is a proposal of a method of detecting at least one kind of disease selected from heart failure, cardiomyopathy, myocarditis, and acute myocardial infarction, involving using, as a marker, any one or more values of a kappa FLC level, a lambda FLC level, and a kappa chain/lambda chain ratio. In Non-patent Literature 1, there is a proposal to use cFLC (polyclonal combined immunoglobulin free light chain: total value of a kappa FLC content and a lambda FLC content) as a marker for a risk of a cardiovascular disease event in a type 2 diabetic patient. In Non-patent Literature 2, it is shown that a kappa chain/lambda chain ratio rises progressively through chronic kidney disease (CKD) stages.

Further, in Patent Literature 1 and Non-patent Literature 3, there is reported a method of detecting a kappa FLC level and a lambda FLC level with higher sensitivity.

### Citation List

### Patent Literature

PTL 1: JP 4950879 B2
PTL 2: WO 2010/049672 A2

### Non-patent Literature

NPL 1: Diabetes care, 2014, 37, p2028-2030
NPL 2: Clin J Am Soc Nephrol. 2008 November; 3(6): 1684-1690.
NPL 3: J Immunol Methods. 2013, May 31; 391(1-2): 1-13

### Summary of Invention

### Technical Problem

Diagnosis of diabetes is currently performed using a blood glucose level, the concentration of hemoglobin A1c, or the like as a marker. However, the blood glucose level fluctuates due to the influence of a meal or the like, and hence a fasting blood glucose level needs to be measured for the diagnosis of diabetes. In addition, after blood collection, red blood cells metabolize glucose in the blood in a blood collection tube, and hence a blood collection tube containing sodium fluoride as a glycolytic inhibitor needs to be used for blood collection for the measurement of the blood glucose level, hemoglobin A1c, or the like.

In view of the foregoing, an object of the present invention is to provide a biomarker for diabetes, which is not affected by a meal or the like, and is hardly metabolized during storage after blood collection. In particular, type I diabetes is an autoimmune disease, and hence another object of the present invention is to provide a method of detecting the presence or absence of activation of immunoglobulin-producing cells in a diabetic patient.

### Solution to Problem

The inventor of the present invention has made extensive investigations, and as a result, has found that, in a diabetic patient, the concentration of a kappa FLC in blood is reduced as compared to that in a healthy subject, and the concentration of a lambda FLC in the blood is increased as compared to that in the healthy subject. The inventor has further found that, along with this phenomenon, in the diabetic patient, the ratio of the concentration of the kappa FLC to the concentration of the lambda FLC in the blood, and the absolute value of a difference between the concentration of the kappa FLC and the concentration of the lambda FLC are markedly reduced in the diabetic patient as compared to those in the healthy subject.

The present invention has been completed on the basis of the findings, and includes the following aspects.

### Item 1.

A method of determining morbidity of diabetes for a test subject, including the following steps (1) to (4), steps (1') to (4'), steps (A) to (D), or steps (A') to (D'):
(1) measuring concentrations of a kappa chain and a lambda chain, which are included in free immunoglobulin light chains, in a specimen collected from a test subject;
(2) calculating a ratio of the concentration of the kappa chain to the concentration of the lambda chain obtained in the step (1) (kappa chain/lambda chain ratio calculated value);
(3) comparing the kappa chain/lambda chain ratio calculated value obtained in the step (2) to a kappa chain/lambda chain ratio defined as a threshhold (kappa chain/lambda chain ratio threshhold value); and
(4) determining that the test subject is suffering from diabetes when, in the step (3), the kappa chain/lambda chain ratio calculated value is lower than the kappa chain/lambda chain ratio threshhold value;
   (1') measuring concentrations of a kappa chain and a lambda chain, which are included in free immunoglobulin light chains, in a specimen collected from a test subject;
   (2') calculating a ratio of the concentration of the lambda chain to the concentration of the kappa chain obtained in the step (1') (lambda chain/kappa chain ratio calculated value);
   (3') comparing the lambda chain/kappa chain ratio calculated value obtained in the step (2') to a lambda chain/kappa chain ratio defined as a threshhold (lambda chain/kappa chain ratio threshhold value); and
   (4') determining that the test subject is suffering from diabetes when, in the step (3'), the lambda chain/kappa chain ratio calculated value is higher than the lambda chain/kappa chain ratio threshhold value;
      (A) measuring concentrations of a kappa chain and a lambda chain, which are included in free immunoglobulin light chains, in a specimen collected from a test subject;
      (B) calculating an absolute value of a difference between the concentration of the kappa chain and the concentration of the lambda chain obtained in the step (A) (kappa chain-lambda chain calculated value);
      (C) comparing the kappa chain-lambda chain calculated value obtained in the step (B) to an absolute value of a difference between a concentration of the kappa chain and a concentration of the lambda chain defined as a threshhold (kappa chain-lambda chain threshhold value); and
      (D) determining that the test subject is suffering from diabetes when, in the step (C), the kappa chain-lambda chain calculated value is lower than the kappa chain-lambda chain threshhold value; or
         (A') measuring concentrations of a kappa chain and a lambda chain, which are included in free immunoglobulin light chains, in a specimen collected from a test subject;
         (B') calculating an absolute value of a difference between the concentration of the lambda chain and the concentration of the kappa chain obtained in the step (A') (lambda chain-kappa chain calculated value);
         (C') comparing the lambda chain-kappa chain calculated value obtained in the step (B') to an absolute value of a difference between a concentration of the lambda chain and a concentration of the kappa chain defined as a threshhold (lambda chain-kappa chain threshhold value); and
         (D') determining that the test subject is suffering from diabetes when, in the step (C'), the lambda chain-kappa chain calculated value is lower than the lambda chain-kappa chain threshhold value.

### Item 2.

A method of determining morbidity of diabetes for a test subject, including the following steps (i) to (iii):
(i) measuring a concentration of a lambda chain, which is included in free immunoglobulin light chains, in a specimen collected from a test subject;
(ii) comparing the concentration of the lambda chain (lambda chain concentration) obtained in the step (i) to a concentration of the lambda chain defined as a threshhold (lambda chain threshhold value); and
(iii) determining that the test subject is suffering from diabetes when the lambda chain concentration is higher than the lambda chain threshhold value.

### Item 3.

A method of determining morbidity of diabetes for a test subject, including the following steps (I) to (III):
(I) measuring a concentration of a kappa chain, which is included in free immunoglobulin light chains, in a specimen collected from a test subject;
(II) comparing the concentration of the kappa chain (kappa chain concentration) obtained in the step (i) to a concentration of the kappa chain defined as a threshhold (kappa chain threshhold value); and
(III) determining that the test subject is suffering from diabetes when the kappa chain concentration is lower than the kappa chain threshhold value.

### Item 4.

A method of detecting a presence or absence of activation of immunoglobulin-producing cells for a diabetic patient, including the following steps (1) to (4), steps (1') to (4'), steps (A) to (D), steps (A') to (D'), or steps (1") to (4") :
(1) measuring concentrations of a kappa chain and a lambda chain, which are included in free immunoglobulin light chains, in a specimen collected from a test subject suffering from diabetes;
(2) calculating a ratio of the concentration of the kappa chain to the concentration of the lambda chain obtained in the step (1) (kappa chain/lambda chain ratio calculated value);
(3) comparing the kappa chain/lambda chain ratio calculated value obtained in the step (2) to a kappa chain/lambda chain ratio defined as a threshhold (kappa chain/lambda chain ratio threshhold value); and
(4) determining that immunoglobulin-producing cells are activated in the test subject when, in the step (3), the kappa chain/lambda chain ratio calculated value is lower than the kappa chain/lambda chain ratio threshhold value;
   (1') measuring concentrations of a kappa chain and a lambda chain, which are included in free immunoglobulin light chains, in a specimen collected from a test subject;
   (2') calculating a ratio of the concentration of the lambda chain to the concentration of the kappa chain obtained in the step (1') (lambda chain/kappa chain ratio calculated value);
   (3') comparing the lambda chain/kappa chain ratio calculated value obtained in the step (2') to a lambda chain/kappa chain ratio defined as a threshhold (lambda chain/kappa chain ratio threshhold value); and
   (4') determining that immunoglobulin-producing cells are activated in the test subject when, in the step (3'), the lambda chain/kappa chain ratio calculated value is higher than the lambda chain/kappa chain ratio threshhold value;
      (A) measuring concentrations of a kappa chain and a lambda chain, which are included in free immunoglobulin light chains, in a specimen collected from a test subject;
      (B) calculating an absolute value of a difference between the concentration of the kappa chain and the concentration of the lambda chain obtained in the step (A) (kappa chain-lambda chain calculated value);
      (C) comparing the kappa chain-lambda chain calculated value obtained in the step (B) to an absolute value of a difference between a concentration of the kappa chain and a concentration of the lambda chain defined as a threshhold (kappa chain-lambda chain threshhold value); and
      (D) determining that immunoglobulin-producing cells are activated in the test subject when, in the step (C), the kappa chain-lambda chain calculated value is lower than the kappa chain-lambda chain threshhold value;
         (A') measuring concentrations of a kappa chain and a lambda chain, which are included in free immunoglobulin light chains, in a specimen collected from a test subject;
         (B') calculating an absolute value of a difference between the concentration of the lambda chain and the concentration of the kappa chain obtained in the step (A') (lambda chain-kappa chain calculated value);
         (C') comparing the lambda chain-kappa chain calculated value obtained in the step (B') to an absolute value of a difference between a concentration of the lambda chain and a concentration of the kappa chain defined as a threshhold (lambda chain-kappa chain threshhold value); and
         (D') determining that immunoglobulin-producing cells are activated in the test subject when, in the step (C'), the lambda chain-kappa chain calculated value is lower than the lambda chain-kappa chain threshhold value; or
            (1") measuring concentrations of a kappa chain and a lambda chain, which are included in free immunoglobulin light chains, in a specimen collected from a test subject suffering from diabetes;
            (2") calculating a sum of the concentration of the kappa chain and the concentration of the lambda chain obtained in the step (1") (kappa chain+lambda chain calculated value);
            (3") comparing the kappa chain+lambda chain calculated value obtained in the step (2") to a kappa chain+lambda chain value defined as a threshhold (kappa chain+lambda chain threshhold value); and
            (4") determining that immunoglobulin-producing cells are activated in the test subject when, in the step (3"), the kappa chain+lambda chain calculated value is higher than the kappa chain+lambda chain threshhold value.

### Item 5.

A method of detecting a presence or absence of activation of immunoglobulin-producing cells for a diabetic patient, including the following steps (i) to (iii):
(i) measuring a concentration of a lambda chain, which is included in free immunoglobulin light chains, in a specimen collected from a test subject suffering from diabetes;
(ii) comparing the concentration of the lambda chain (lambda chain concentration) obtained in the step (i) to a concentration of the lambda chain defined as a threshhold (lambda chain threshhold value); and
(iii) determining that immunoglobulin-producing cells are activated in the test subject when the lambda chain concentration is higher than the lambda chain threshhold value.

### Item 6.

A method of detecting a presence or absence of activation of immunoglobulin-producing cells for a diabetic patient, including the following steps (I) to (III):
(I) measuring a concentration of a kappa chain, which is included in free immunoglobulin light chains, in a specimen collected from a test subject suffering from diabetes;
(II) comparing the concentration of the kappa chain (kappa chain concentration) obtained in the step (I) to a concentration of the kappa chain defined as a threshhold (kappa chain threshhold value); and
(III) determining that immunoglobulin-producing cells are activated in the test subject when the kappa chain concentration is lower than the kappa chain threshhold value.

### Item 7.

A test kit for carrying out the method of Item 1 or 4, including:
a free immunoglobulin kappa light chain capture antibody;
a free immunoglobulin kappa light chain detection antibody;
a free immunoglobulin lambda light chain capture antibody; and
a free immunoglobulin lambda light chain detection antibody.

### Item 8.

A test kit for carrying out the method of Item 1, 2, 4, or 5, including:
a free immunoglobulin lambda light chain capture antibody; and
a free immunoglobulin lambda light chain detection antibody.

### Item 9.

A test kit for carrying out the method of Item 1, 3, 4, or 6, including:
a free immunoglobulin kappa light chain capture antibody; and
a free immunoglobulin kappa light chain detection antibody.

### Item 10.

A test reagent for carrying out the method of Item 1, 2, 4, or 5, including a free immunoglobulin lambda light chain antibody.

### Item 11.

A test reagent for carrying out the method of Item 1, 3, 4, or 6, including a free immunoglobulin kappa light chain antibody.

### Advantageous Effects of Invention

According to the present invention, the morbidity of diabetes can be determined using serum or plasma to be used for another biochemical test or the like. In addition, blood collection for the measurement does not need to be performed at the time of fasting, and blood collection using a special anticoagulant does not need to be performed. Accordingly, a burden on a patient at the time of the blood collection can be relieved. In addition, according to the present invention, the activation of immunoglobulin-producing cells can be detected for a diabetic patient. As a result, it can be judged that the diabetic patient is accompanied by the activation of immunoglobulin-producing cells, and hence it can be judged whether treatment for suppressing the activation of immunoglobulin-producing cells needs to be performed for the diabetic patient.

### Brief Description of Drawings

FIG. 1 is a graph for showing a distribution of kappa FLC/lambda FLC ratio calculated values of diabetic patients and healthy subjects.
FIG. 2 is a graph for showing a distribution of kappa FLC-lambda FLC calculated values of the diabetic patients and the healthy subjects.
FIG. 3 is a graph for showing a distribution of kappa FLC concentrations of the diabetic patients and the healthy subjects.
FIG. 4 is a graph for showing a distribution of lambda FLC concentrations of the diabetic patients and the healthy subjects.
FIG. 5 is a graph for showing a distribution of kappa FLC+lambda FLC ratio calculated values of the diabetic patients and the healthy subjects.
FIG. 6 is a graph for showing an ROC curve of the kappa FLC/lambda FLC ratio calculated values of the diabetic patients and the kappa FLC/lambda FLC ratio calculated values of the healthy subjects.
FIG. 7 is a graph for showing an ROC curve of the kappa FLC-lambda FLC calculated values of the diabetic patients and the kappa FLC-lambda FLC calculated values of the healthy subjects.
FIG. 8 is a graph for showing an ROC curve of the kappa FLC concentrations of the diabetic patients and the kappa FLC concentrations of the healthy subjects.
FIG. 9 is a graph for showing an ROC curve of the lambda FLC concentrations of the diabetic patients and the lambda FLC concentrations of the healthy subjects.
FIG. 10 is a graph for showing an ROC curve of the kappa FLC+lambda FLC ratio calculated values of the diabetic patients and the kappa FLC+lambda FLC ratio calculated values of the healthy subjects.

### Description of Embodiments

### 1. Description of Terms

First, terms used in this description, the claims, and the abstract in the context of the present invention are described.

In the present invention, the term "diabetes" refers to, in the case of a human, the "diabetes mellitus" described in "Report of the Committee on the Classification and Diagnostic Criteria of Diabetes Mellitus (Revision for International Harmonization of HbA1c in Japan)" published in "Tonyobyo", the Journal of The Japan Diabetes Society (Vol. 55 (2012) No. 7 p. 485-504: hereinafter referred to as "Committee Report"). Specifically, whether one has diabetes is determined in accordance with Table 1 (citing Table 4 of the Committee Report).

**Table 1**

| |
|---|
| Clinical diagnosis: |
| 1) A case in which, in an initial test, any one of (1) fasting blood glucose level≥126 mg/dl, (2) 2-hour 75 g OGTT value≥200 mg/dl, (3) casual blood glucose level≥200 mg/dl, and (4) HbA1c (NGSP)≥6.5% (HbA1c (JDS)≥6.1%) is found is judged to be a "diabetic type". A retest is performed on another day, and if the "diabetic type" is confirmed again, the case is diagnosed as diabetes*. However, diagnosis by repeated tests of only HbA1c is not allowed. In addition, if it is confirmed that the blood glucose level and HbA1c indicate the diabetic type in the same blood collection (any one of (1) to (3), and (4)), the case may be diagnosed as diabetes by the initial test alone. |
| 2) A case in which the blood glucose level indicates the diabetic type (any one of (1) to (3)), and any one of the following conditions is satisfied may be diagnosed as diabetes by the initial test alone. |
| • Presence of typical symptoms of diabetes (dry mouth, polydipsia, polyuria, and weight loss) |
| • Presence of definite diabetic retinopathy |
| 3) A case in which it can be confirmed that the condition of 1) or 2) above was satisfied in the past needs to be diagnosed as diabetes or dealt with as suspected diabetes, even if the current test value does not meet the above-mentioned condition. |
| 4) In a case that is difficult to judge as diabetes even by 1) to 3) above, the patient is followed up with suspicion of diabetes and is retested after a period of time. |
| 5) Please note the following in selecting a judgment method in the initial test and the retest. |
| • When HbA1c is used for the judgment in the initial test, it is essential for diagnosis to include another judgment method in the retest. In the testing, in principle, both the blood glucose level and HbA1c are measured. |
| • When the judgment in the initial test is performed on the basis of casual blood glucose level≥200 mg/dl, it is desired for the retest to be performed by another test method. |
| • In the case of a disease/situation in which HbA1c and an average blood glucose level may have a gap, diagnosis based on the blood glucose level is required (Table 5). |
| Epidemiological survey: In the case of aiming to estimate the frequency of diabetes, the judgment of the "diabetic type" by a single test may be read as "diabetes". The criterion of HbA1c(NGSP)≥6.5% (HbA1c(JDS)≥6.1%) or 2-hour OGTT value≥200 mg/dl is used if possible. |
| Examination: It is important to surely detect diabetes and its high risk group. For screening, not only the blood glucose level and HbA1c, but also clinical information, such as family history and obesity, is referred to. |

| |
|---|
| *Confirmation of hyperglycemia in a stress-free state is required. |

In the present invention, the term "diabetic patient" refers to a patient diagnosed as suffering from diabetes, and a patient who has developed diabetic nephropathy may be preferably excluded from the diabetic patient.

In the present invention, the term "test subject" refers to an individual to which a determination method according to the present invention to be described later is applicable.

In the present invention, the "individual" is not particularly limited, and examples thereof include mammals, such as a human, a mouse, a rat, a dog, a cat, a rabbit, a bovine, a horse, a goat, a sheep, and a pig. Of those, a human, a dog, a cat, or the like is preferred, and a human is more preferred. In addition, the individual encompasses both of an individual having a disease and an individual having no disease (healthy individual). The age and gender (male or female) of the individual are not limited.

In the present invention, the term "specimen" refers to cells, a tissue, a blood sample, and body fluids (sweat, a secretion from a skin, tears, saliva, cerebrospinal fluid, ascites, and pleural effusion) derived from the test subject, preferably urine and a blood sample, more preferably a blood sample. The "blood sample" encompasses, for example, blood (whole blood) collected from the test subject, or serum or plasma prepared from the blood. Of those, serum or plasma is more preferred, and serum is still more preferred. The kind of an anticoagulant to be used for collecting plasma is not particularly limited. The blood sample of the test subject to be used for measurement and a blood sample for determining a threshhold value may be of the same kind or may be of different kinds, but are preferably of the same kind. In addition, when plasma is used as the blood sample, plasma for determining the threshhold value is preferably prepared from blood collected using the same anticoagulant as the plasma of the test subject.

Further, the specimen may be fresh, or may be stored. When the specimen is stored, the specimen may be stored in a room temperature environment, a refrigerated environment, or a frozen environment, and frozen storage is preferred.

In the present invention, immunoglobulin-producing cells refer to cells capable of producing immunoglobulins, and include B lymphocytes, plasma cells, and the like. In addition, in the present invention, activation of immunoglobulin-producing cells refers to a state in which the antibody-producing ability of the immunoglobulin-producing cells is enhanced or can be enhanced.

In the present invention, a free immunoglobulin light chain (FLC) is also referred to as immunoglobulin free L chain or Bence Jones protein. The free immunoglobulin light chain is, for example, a monomer or dimer of an immunoglobulin light chain not associated with an immunoglobulin heavy chain. Immunoglobulin light chains are classified into two kinds, i.e., a kappa (κ) chain and a lambda (λ) chain on the basis of a difference in antigenicity. Hereinafter, the free immunoglobulin kappa light chain is sometimes referred to as kappa FLC, or simply as kappa chain. Similarly, the free immunoglobulin lambda light chain is sometimes referred to as lambda FLC, or simply as lambda chain.

The "concentration of the kappa chain" refers to the concentration of the free immunoglobulin kappa light chain in the specimen, and may be a molar concentration or may be a ratio of mass per given volume of the specimen (mass/volume), but is preferably the mass/volume. The "concentration of the lambda chain" refers to the concentration of the free immunoglobulin lambda light chain in the specimen, and may be a molar concentration or may be a ratio of mass per given volume of the specimen (mass/volume), but is preferably the mass/volume.

As a value reflecting the "concentration", in addition to the foregoing, the intensity of a signal, such as fluorescence or luminescence, may be used.

In the present invention, the ratio of the concentration of the kappa chain to the concentration of the lambda chain is sometimes referred to as "kappa chain/lambda chain ratio calculated value", and the ratio of the concentration of the lambda chain to the concentration of the kappa chain is sometimes referred to as "lambda chain/kappa chain ratio calculated value". In addition, the absolute value of a difference between the concentration of the kappa chain and the concentration of the lambda chain is sometimes referred to as "kappa chain-lambda chain calculated value", and the absolute value of a difference between the concentration of the lambda chain and the concentration of the kappa chain is sometimes referred to as "lambda chain-kappa chain calculated value". The symbol "-" means "minus". Further, the concentration of the kappa chain is sometimes referred to as "kappa chain concentration", and the concentration of the lambda chain is sometimes referred to as "lambda chain concentration". In addition, the sum of the concentration of the kappa chain and the concentration of the lambda chain is sometimes referred to as "kappa chain+lambda chain calculated value". The symbol "+" means "plus".

In the present invention, the term "threshhold value" refers to, for example, a predetermined threshhold value obtained from the concentration of the kappa chain and/or the lambda chain in a specimen from an individual not suffering from diabetes, and is preferably a predetermined threshhold value obtained from the concentration of the kappa chain and/or the lambda chain in a specimen from a healthy individual.

For example, the "predetermined threshhold value" is as follows: In "3. Method of determining Morbidity of Diabetes" and "4. Method of detecting Presence or Absence of Activation of Immunoglobulin-producing Cells" to be described later, a threshhold value (kappa chain/lambda chain ratio threshhold value) corresponding to the ratio of the concentration of the kappa chain to the concentration of the lambda chain (kappa chain/lambda chain ratio calculated value) in a specimen from a test subject is the ratio of the concentration of the kappa chain to the concentration of the lambda chain in a specimen from an individual not suffering from diabetes; and a threshhold value (lambda chain/kappa chain ratio threshhold value) corresponding to the ratio of the concentration of the lambda chain to the concentration of the kappa chain (lambda chain/kappa chain ratio calculated value) is the ratio of the concentration of the lambda chain to the concentration of the kappa chain in a specimen from an individual not suffering from diabetes. In addition, a threshhold value (kappa chain-lambda chain threshhold value) corresponding to the absolute value of a difference between the concentration of the kappa chain and the concentration of the lambda chain (kappa chain-lambda chain calculated value) in a specimen from a test subject is the absolute value of a difference between the concentration of the kappa chain and the concentration of the lambda chain in a specimen from an individual not suffering from diabetes; and a threshhold value (lambda chain-kappa chain threshhold value) corresponding to the absolute value of a difference between the concentration of the lambda chain and the concentration of the kappa chain (lambda chain-kappa chain calculated value) is the absolute value of a difference between the concentration of the lambda chain and the concentration of the kappa chain in a specimen from an individual not suffering from diabetes. Moreover, a threshhold value (kappa chain+lambda chain threshhold value) corresponding to the sum of the concentration of the kappa chain and the concentration of the lambda chain (kappa chain+lambda chain calculated value) in a specimen from a test subject is the sum of the concentration of the kappa chain and the concentration of the lambda chain in a specimen from an individual not suffering from diabetes. Further, a threshhold value (kappa chain threshhold value) corresponding to the concentration of the kappa chain (kappa chain concentration) in a specimen from a test subject is the concentration of the kappa chain in a specimen from an individual not suffering from diabetes. Besides, a threshhold value (lambda chain threshhold value) corresponding to the concentration of the lambda chain (lambda chain concentration) in a specimen from a test subject is the concentration of the lambda chain in a specimen from an individual not suffering from diabetes.

In addition, as another aspect of threshhold value of the "predetermined threshhold value", the threshhold value may be obtained in the following manner. For example, kappa chain/lambda chain ratio calculated values, lambda chain/kappa chain ratio calculated values, kappa chain-lambda chain calculated values, lambda chain-kappa chain calculated values, or kappa chain+lambda chain calculated values, which are calculated from specimens collected from a plurality of individuals suffering from diabetes, and kappa chain/lambda chain ratio calculated values, lambda chain/kappa chain ratio calculated values, kappa chain-lambda chain calculated values, lambda chain-kappa chain calculated values, or kappa chain+lambda chain calculated values, which are calculated from specimens from a plurality of individuals not suffering from diabetes, are obtained. On the basis of the plurality of values, a value that can most accurately separate a diabetes positive (hereinafter referred to simply as "positive") and a diabetes negative (hereinafter referred to simply as "negative") may be adopted as the "threshhold value". Specimens collected from the plurality of individuals suffering from diabetes are each measured for the concentration of the kappa chain or the concentration of the lambda chain, and specimens from the plurality of individuals not suffering from diabetes are each measured for the concentration of the kappa chain or the concentration of the lambda chain. On the basis of the resultant plurality of values, a value that can most accurately separate the "positive" and the "negative" may be adopted as the "threshhold value". Herein, the "value that can most accurately separate" may be appropriately set on the basis of a value, such as sensitivity, specificity, a positive predictive value, or a negative predictive value, depending on the purpose of testing.

In addition, another aspect of the "predetermined threshhold value" is as described below.

As one aspect, when diabetes is diagnosed using the kappa chain/lambda chain ratio calculated value as a marker, the highest value among kappa chain/lambda chain ratio calculated values in respective specimens obtained from a plurality of individuals suffering from diabetes may be adopted as the threshhold value. For example, when it is desired to reduce false negatives to the extent possible as in a screening test, such threshhold value may be suitably used. Conversely, when diabetes is diagnosed using the kappa chain/lambda chain ratio calculated value as a marker, the lowest value among kappa chain/lambda chain ratio calculated values in specimens from a plurality of individuals not suffering from diabetes may be determined as the threshhold value. For example, when it is desired to reduce false positives to the extent possible, such threshhold value may be suitably used.

As one aspect, when diabetes is diagnosed using the lambda chain/kappa chain ratio calculated value as a marker, the lowest value among lambda chain/kappa chain ratio calculated values in respective specimens obtained from a plurality of individuals suffering from diabetes may be adopted as the threshhold value. For example, when it is desired to reduce false positives to the extent possible as in a screening test, such threshhold value may be suitably used. Conversely, when diabetes is diagnosed using the lambda chain/kappa chain ratio calculated value as a marker, the highest value among lambda chain/kappa chain ratio calculated values in specimens from a plurality of individuals not suffering from diabetes may be determined as the threshhold value. For example, when it is desired to reduce false negatives to the extent possible, such threshhold value may be suitably used.

As another aspect of the "predetermined threshhold value", when diabetes is diagnosed using the kappa chain-lambda chain calculated value as a marker, the highest value among kappa chain-lambda chain calculated values in respective specimens obtained from a plurality of individuals suffering from diabetes may be adopted as the threshhold value. For example, when it is desired to reduce false negatives to the extent possible as in a screening test, such threshhold value may be suitably used. Conversely, when diabetes is diagnosed using the kappa chain-lambda chain calculated value as a marker, the lowest value among kappa chain-lambda chain calculated values in specimens from a plurality of individuals not suffering from diabetes may be determined as the threshhold value. For example, when it is desired to reduce false positives to the extent possible, such threshhold value may be suitably used.

As another aspect of the "predetermined threshhold value", when diabetes is diagnosed using the lambda chain-kappa chain calculated value as a marker, the highest value among lambda chain-kappa chain calculated values in respective specimens obtained from a plurality of individuals suffering from diabetes may be adopted as the threshhold value. For example, when it is desired to reduce false negatives to the extent possible as in a screening test, such threshhold value may be suitably used. Conversely, when diabetes is diagnosed using the lambda chain-kappa chain calculated value as a marker, the lowest value among lambda chain-kappa chain calculated values in specimens from a plurality of individuals not suffering from diabetes may be determined as the threshhold value. For example, when it is desired to reduce false positives to the extent possible, such threshhold value may be suitably used.

As another aspect of the "predetermined threshhold value", when diabetes is diagnosed using the kappa chain+lambda chain ratio calculated value as a marker, the lowest value among kappa chain+lambda chain ratio calculated values in respective specimens obtained from a plurality of individuals suffering from diabetes may be adopted as the threshhold value. For example, when it is desired to reduce false positives to the extent possible as in a screening test, such threshhold value may be suitably used. Conversely, when diabetes is diagnosed using the kappa chain+lambda chain ratio calculated value as a marker, the highest value among kappa chain+lambda chain ratio calculated values in specimens from a plurality of individuals not suffering from diabetes may be determined as the threshhold value. For example, when it is desired to reduce positives to the extent possible, such threshhold value may be suitably used.

As another aspect of the "predetermined threshhold value", when diabetes is diagnosed using the kappa chain concentration as a marker, the highest value among kappa chain concentrations in respective specimens obtained from a plurality of individuals suffering from diabetes may be adopted as the threshhold value. For example, when it is desired to reduce false negatives to the extent possible as in a screening test, such threshhold value may be suitably used. Conversely, when diabetes is diagnosed using the kappa chain concentration as a marker, the lowest value among kappa chain concentrations in specimens from a plurality of individuals not suffering from diabetes may be determined as the threshhold value. For example, when it is desired to reduce false positives to the extent possible, such threshhold value may be suitably used.

As another aspect of the "predetermined threshhold value", when diabetes is diagnosed using the lambda chain concentration as a marker, the lowest value among lambda chain concentrations in respective specimens obtained from a plurality of individuals suffering from diabetes may be adopted as the threshhold value. For example, when it is desired to reduce false positives to the extent possible as in a screening test, such threshhold value may be suitably used. Conversely, when diabetes is diagnosed using the lambda chain concentration as a marker, the highest value among lambda chain concentrations in specimens from a plurality of individuals not suffering from diabetes may be determined as the threshhold value. For example, when it is desired to reduce false negatives to the extent possible, such threshhold value may be suitably used.

In addition, as another aspect, the threshhold value may be the kappa chain/lambda chain ratio calculated value, kappa chain-lambda chain calculated value, kappa chain+lambda chain ratio calculated value, kappa chain concentration, or lambda chain concentration itself in a specimen from an individual not suffering from diabetes, or the mean, median, or aspect of kappa chain/lambda chain ratio calculated values, kappa chain-lambda chain calculated values, kappa chain+lambda chain ratio calculated values, kappa chain concentrations, or lambda chain concentrations in specimens from a plurality of individuals not suffering from diabetes.

Besides, as the threshhold value, the kappa chain/lambda chain ratio calculated value, lambda chain/kappa chain ratio calculated value, kappa chain-lambda chain calculated value, lambda chain-kappa chain calculated value, kappa chain+lambda chain ratio calculated value, kappa chain concentration, or lambda chain concentration (which may be one value, or may be, for example, the mean, median, or aspect of a plurality of values) of a test subject before being suffering from diabetes may be used for the same test subject.

As still another aspect, when the above-mentioned threshhold value is determined, the kappa chain/lambda chain ratio calculated value, kappa chain-lambda chain calculated value, kappa chain+lambda chain ratio calculated value, kappa chain concentration, or lambda chain concentration in a specimen from a healthy individual may be used in place of the kappa chain/lambda chain ratio calculated value, lambda chain/kappa chain ratio calculated value, kappa chain-lambda chain calculated value, lambda chain-kappa chain calculated value, kappa chain+lambda chain ratio calculated value, kappa chain concentration, or lambda chain concentration in a specimen from an individual not suffering from diabetes.

Those threshhold values may be determined at the time of obtaining the kappa chain/lambda chain ratio calculated value, lambda chain/kappa chain ratio calculated value, kappa chain-lambda chain calculated value, lambda chain-kappa chain calculated value, kappa chain+lambda chain ratio calculated value, kappa chain concentration, or lambda chain concentration in the specimen from the test subject, but may be determined in advance.

The "healthy individual" is not particularly limited. The "healthy individual" preferably refers to an individual that is a human or non-human mammal described in the section for the "individual", and that does not show abnormal data in a biochemical test, a blood test, a urine test, a serum test, a physiological test, and the like. The age and gender of the healthy individual are not particularly limited.

The "individual not suffering from diabetes" is not particularly limited except that the individual is not suffering from diabetes. The "individual not suffering from diabetes" preferably refers to an individual that is a human or non-human mammal described in the section for the "individual", and that cannot be diagnosed with diabetes in accordance with known diagnostic criteria.

The "plurality of specimens" refers to two or more, preferably five or more, more preferably ten or more biological samples. The "plurality of specimens" may be specimens collected from different individuals, or may be a plurality of specimens from the same individual collected at different times.

The "plurality of values" refers to two or more, preferably five or more, more preferably ten or more kappa chain/lambda chain ratio calculated values, lambda chain/kappa chain ratio calculated values, kappa chain-lambda chain calculated values, lambda chain-kappa chain calculated values, kappa chain+lambda chain ratio calculated values, kappa chain concentrations, or lambda chain concentrations.

The "plurality of individuals" refers to two or more, preferably five or more, more preferably ten or more individuals.

An individual from which a kappa chain/lambda chain ratio calculated value, lambda chain/kappa chain ratio calculated value, kappa chain-lambda chain calculated value, lambda chain-kappa chain calculated value, kappa chain+lambda chain ratio calculated value, kappa chain concentration, or lambda chain concentration is obtained in order to determine the threshhold value, and the test subject are not necessarily required to be of the same species, age, gender, or the like, but are preferably of the same kind. In addition, the individual is preferably of the same generation and/or the same gender as the test subject.

In the present invention, a "free immunoglobulin kappa light chain antibody" is also referred to as "anti-kappa chain antibody". The free immunoglobulin kappa light chain antibody is not limited as long as the free immunoglobulin kappa light chain antibody specifically binds to the kappa chain, and any of polyclonal antibodies and monoclonal antibodies obtained by immunizing a non-human animal with the kappa chain or part thereof used as an antigen, and fragments thereof (e.g., Fab and F(ab)₂) may be used. However, it is preferred to remove at least a hypervariable region from the antigen. In addition, the class and subclass of the immunoglobulin are not particularly limited. In addition, the anti-kappa chain antibody may be a chimeric antibody. Further, the anti-kappa chain antibody may be an scFv or the like. In addition, as the anti-kappa chain antibody, there may also be used an antibody described in, for example, Patent Literature 2 or Non-patent Literature 3, or a commercially available product, such as Anti-Kappa light chain antibody [RM103] (ab190484) (Abcam plc).

In the present invention, a "free immunoglobulin lambda light chain antibody" is also referred to as "anti-lambda chain antibody". The free immunoglobulin lambda light chain antibody is not limited as long as the free immunoglobulin lambda light chain antibody specifically binds to the lambda chain, and any of polyclonal antibodies and monoclonal antibodies obtained by immunizing a non-human animal with the kappa chain or part thereof used as an antigen, and fragments thereof (e.g., Fab and F(ab)₂) may be used. However, it is preferred to remove at least a hypervariable region from the antigen. In addition, the class and subclass of the immunoglobulin are not particularly limited. In addition, the anti-lambda chain antibody may be a chimeric antibody. Further, the anti-lambda chain antibody may be an scFv or the like. In addition, as the anti-lambda chain antibody, there may also be used an antibody described in, for example, Patent Literature 2 or Non-patent Literature 3, or a commercially available product, such as Anti-Lambda Light chain antibody [2G9] (ab1943) (Abcam plc).

In the present invention, the term "capture antibody" refers to an antibody to be used for the purpose of capturing an antigen in an ELISA method or the like. In addition, the term "detection antibody" refers to an antibody to be used for the purpose of detecting an antigen, preferably an antibody labeled with a labeling substance as described later.

### 2. Method of measuring Concentrations of Kappa FLC and Lambda FLC

In this section, description is made of a method of measuring the concentrations of a kappa FLC and a lambda FLC in "3. Method of determining Morbidity of Diabetes" and "4. Method of detecting Presence or Absence of Activation of Immunoglobulin-producing Cells" to be described later.

When the concentrations of the kappa FLC and the lambda FLC are measured, a measurement method using the anti-kappa chain antibody or the anti-lambda chain antibody described in the "1. Description of Terms" may be used. A known ELISA method or the like may be used as the method of measuring the concentration of the kappa chain or the concentration of the lambda chain. In addition, the measurement may be performed using a competitive ELISA method as a method with higher sensitivity. The concentrations of the kappa FLC and the lambda FLC may be measured in two independent measurement systems or in one measurement system.

In this aspect, the anti-kappa chain antibody or anti-lambda chain antibody for antigen capture may be immobilized in advance onto a solid phase, such as a microplate, fluorescent beads, or magnetic beads, to form a complex of the immobilized anti-kappa chain antibody or anti-lambda chain antibody and the kappa chain or lambda chain in the specimen. Through the detection of the complex immobilized onto the solid phase or the complex formed on the solid phase by a method known in the art, the concentration of the kappa chain contained in the specimen or the concentration of the lambda chain contained therein may be measured. In addition, in this method, the complex of the anti-kappa chain antibody for antigen capture or the anti-lambda chain antibody for antigen capture and the kappa chain or lambda chain in the specimen may be formed prior to the immobilization of the complex to the solid phase.

A method of immobilizing the anti-kappa chain antibody or anti-lambda chain antibody for antigen capture to the solid phase is not particularly limited. The immobilization may be performed directly or indirectly via another substance, through the use of a known method. As direct binding, for example, physical adsorption is given. Specifically, through the use of an immunoplate or the like, each capture antibody may be physically bound directly to a microplate. In addition, the anti-kappa chain antibody or anti-lambda chain antibody for antigen capture may be indirectly immobilized to the solid phase. Indirect immobilization of the capture antibody to fluorescent beads, magnetic beads, or the like may be performed in accordance with a method described in, for example, Patent Literature 2 or Non-patent Literature 3.

The shape of the solid phase is not particularly limited, and examples thereof include a microplate, a microtube, a test tube, and beads. A material for the solid phase is not particularly limited, and for example, polystyrene, polypropylene, or the like may be used for the microplate, the microtube, the test tube, or the like. In addition, in the case of the beads, polystyrene Xmap (trademark) beads (Luminex Corporation), MagPlex (trademark) Microspheres (Luminex Corporation), or the like may be used.

This method may include an operation of washing the solid phase subsequent to the formation of the complex. When the washing is performed, PBS or the like containing a surfactant or the like may be used.

In this method, the complex may be detected, in the case of a general ELISA method, by using an anti-kappa chain antibody for detection labeled with a labeling substance or an anti-lambda chain antibody for detection labeled with a labeling substance, or by using an anti-immunoglobulin antibody or the like labeled with a labeling substance capable of binding to an unlabeled anti-kappa chain antibody or an unlabeled anti-lambda chain antibody, but it is preferred to use the labeled anti-kappa chain antibody for detection or the labeled anti-lambda chain antibody for detection. In addition, it is preferred that an epitope in an antigen for the anti-kappa chain antibody or anti-lambda chain antibody to be used for detection be different from an epitope in an antigen for the anti-kappa chain antibody or anti-lambda chain antibody to be used for antigen capture.

When the concentration of the kappa FLC or the lambda FLC is measured by a competitive ELISA method, the kappa FLC or lambda FLC in the specimen may be brought into contact with the anti-kappa chain antibody or anti-lambda chain antibody for capture in the presence of a competitor labeled with a labeling substance. The competitor is a peptide or protein having an epitope to be recognized by the same capture antibody as the kappa FLC or lambda FLC in the specimen. The competitor may be isolated from an individual, or may be synthesized by a genetic engineering approach or a chemical approach.

The labeling substance for labeling the anti-kappa chain antibody or anti-lambda chain antibody to be used for detection, the labeled anti-immunoglobulin antibody, or the competitor is not particularly limited as long as a detectable signal is generated. Examples thereof include a fluorescent substance, a radioactive isotope, and an enzyme. Examples of the enzyme include alkaline phosphatase and peroxidase. Examples of the fluorescent substance include: fluorescent dyes, such as fluorescein isothiocyanate (FITC), rhodamine, and Alexa Fluor (trademark); and fluorescent proteins, such as GFP. Examples of the radioactive isotope include ¹²⁵I, ¹⁴C, and ³²P. Of those, alkaline phosphatase or peroxidase is preferred as the labeling substance.

The anti-kappa chain antibody or anti-lambda chain antibody to be used for detection is obtained by labeling each antibody with the above-mentioned labeling substance by a labeling method known in the art. In addition, the labeling may be performed using a commercially available labeling kit or the like. In addition, for the labeled immunoglobulin antibody, the same technique as that for the labeling of the anti-kappa chain antibody or the anti-lambda chain antibody may be used, or a commercially available product may be used.

In this method, the concentration of the kappa FLC or lambda FLC contained in the specimen may be measured by detecting a signal generated by the labeling substance of the detection antibody contained in the complex. Herein, to "detect the signal" includes qualitatively detecting the presence or absence of the signal, quantifying the intensity of the signal, and semi-quantitatively detecting the intensity of the signal. The semiquantitative detection means indicating the intensity of the signal in stages, such as "no signal generation", "weak", "medium", and "strong". In this method, it is preferred that the intensity of the signal be quantitatively or semi-quantitatively detected.

A known method may be used as a method of detecting the signal. In this method, a measurement method depending on the kind of the signal derived from the above-mentioned labeling substance may be appropriately selected. For example, when the labeling substance is an enzyme, the detection may be performed by measuring a signal, such as light or a color, generated by allowing a substrate for the enzyme to react using a known apparatus, such as a fluorometer, a luminometer, or a spectrophotometer.

The substrate for the enzyme may be appropriately selected from known substrates depending on the kind of the enzyme. For example, when alkaline phosphatase is used as the enzyme, examples of the substrate include: chemiluminescent substrates, such as CDP-Star (trademark) (disodium 4-chloro-3-(methoxyspiro[1,2-dioxetane-3,2'-(5'-chloro)tricyclo[3.3.1.13,7]decan]-4-yl)phenyl phosphate); and chromogenic substrates, such as 5-bromo-4-chloro-3-indolyl phosphate (BCIP), disodium 5-bromo-6-chloro-indolyl phosphate, and p-nitrophenyl phosphate. When the labeling substance is peroxidase, an example thereof is tetramethylbenzidine (TMB).

When the labeling substance is a radioactive isotope, a radiation used as the signal may be measured using a known apparatus, such as a scintillation counter. In addition, when the labeling substance is a fluorescent substance, fluorescence as the signal may be measured using a known apparatus, such as a fluorescence microplate reader or a Luminex (trademark) system (Luminex Corporation). An excitation wavelength and a fluorescence wavelength may be appropriately decided depending on the kind of the fluorescent substance used.

The detection result of the signal may be used as a value for the concentration of the kappa chain or a value for the concentration of the lambda chain. For example, when the intensity of the signal is quantitatively detected, a measured value for the signal intensity itself or a value calculated from the measured value for the signal intensity may be used as the value for the concentration of the kappa chain or the value for the concentration of the lambda chain.

### 3. Method of determining Morbidity of Diabetes

The present invention relates to a method of determining morbidity of diabetes for a test subject. The method of determining morbidity of diabetes includes the following four aspects.

A first aspect includes the following steps (1) to (4): (1) measuring concentrations of a kappa chain and a lambda chain, which are included in free immunoglobulin light chains, in a specimen collected from a test subject, in accordance with the "2. Method of measuring Concentrations of Kappa FLC and Lambda FLC"; (2) calculating a ratio of the concentration of the kappa chain to the concentration of the lambda chain obtained in the step (1) (kappa chain/lambda chain ratio calculated value); (3) comparing the kappa chain/lambda chain ratio calculated value obtained in the step (2) to a kappa chain/lambda chain ratio defined as a threshhold (kappa chain/lambda chain ratio threshhold value); and (4) determining that the test subject is suffering from diabetes when, in the step (3), the kappa chain/lambda chain ratio calculated value is lower than the kappa chain/lambda chain ratio threshhold value.

A second aspect includes the following steps (1') to (4') :
(1') measuring concentrations of a kappa chain and a lambda chain, which are included in free immunoglobulin light chains, in a specimen collected from a test subject, in accordance with the "2. Method of measuring Concentrations of Kappa FLC and Lambda FLC"; (2') calculating a ratio of the concentration of the lambda chain to the concentration of the kappa chain obtained in the step (1') (lambda chain/kappa chain ratio calculated value); (3') comparing the lambda chain/kappa chain ratio calculated value obtained in the step (2') to a lambda chain/kappa chain ratio defined as a threshhold (lambda chain/kappa chain ratio threshhold value); and (4') determining that the test subject is suffering from diabetes when, in the step (3'), the lambda chain/kappa chain ratio calculated value is higher than the lambda chain/kappa chain ratio threshhold value.

A third aspect includes the following steps (A) to (D): (A) measuring concentrations of a kappa chain and a lambda chain, which are included in free immunoglobulin light chains, in a specimen collected from a test subject; (B) calculating an absolute value of a difference between the concentration of the kappa chain and the concentration of the lambda chain obtained in the step (A) (kappa chain-lambda chain calculated value); (C) comparing the kappa chain-lambda chain calculated value obtained in the step (B) to an absolute value of a difference between a concentration of the kappa chain and a concentration of the lambda chain defined as a threshhold (kappa chain-lambda chain threshhold value); and (D) determining that the test subject is suffering from diabetes when, in the step (C), the kappa chain-lambda chain calculated value is lower than the kappa chain-lambda chain threshhold value.

A fourth aspect includes the following steps (A') to (D') :
(A') measuring concentrations of a kappa chain and a lambda chain, which are included in free immunoglobulin light chains, in a specimen collected from a test subject, in accordance with the "2. Method of measuring Concentrations of Kappa FLC and Lambda FLC"; (B') calculating an absolute value of a difference between the concentration of the lambda chain and the concentration of the kappa chain obtained in the step (A') (lambda chain-kappa chain calculated value); (C') comparing the lambda chain-kappa chain calculated value obtained in the step (B') to an absolute value of a difference between a concentration of the lambda chain and a concentration of the kappa chain defined as a threshhold (lambda chain-kappa chain threshhold value); and (D') determining that the test subject is suffering from diabetes when, in the step (C'), the lambda chain-kappa chain calculated value is lower than the lambda chain-kappa chain threshhold value.

A fifth aspect includes the following steps (i) to (iii):
(i) measuring a concentration of a lambda chain, which is included in free immunoglobulin light chains, in a specimen collected from a test subject, in accordance with the "2. Method of measuring Concentrations of Kappa FLC and Lambda FLC"; (ii) comparing the concentration of the lambda chain (lambda chain concentration) obtained in the step (i) to a concentration of the lambda chain defined as a threshhold (lambda chain threshhold value); and (iii) determining that the test subject is suffering from diabetes when the lambda chain concentration is higher than the lambda chain threshhold value.

A sixth aspect includes the following steps (I) to (III) :
(I) measuring a concentration of a kappa chain, which is included in free immunoglobulin light chains, in a specimen collected from a test subject, in accordance with the "2. Method of measuring Concentrations of Kappa FLC and Lambda FLC"; (II) comparing the concentration of the kappa chain (kappa chain concentration) obtained in the step (I) to a concentration of the kappa chain defined as a threshhold (kappa chain threshhold value); and (III) determining that the test subject is suffering from diabetes when the kappa chain concentration is lower than the kappa chain threshhold value.

The first to sixth aspects are based on the following fact: in the diabetic patient, the concentration of the kappa FLC in the specimen is reduced as compared to that in a healthy subject, and the concentration of the lambda FLC in blood is increased as compared to that in the healthy subject. Therefore, the first to sixth aspects may each be carried out alone, but two or more kinds selected from the group consisting of the first to sixth aspects may be carried out in combination. In addition, of the above-mentioned aspects, as the method of determining morbidity of diabetes, the first aspect, the third aspect, the fifth aspect, or the sixth aspect is preferred, and the first aspect, the third aspect, or the fifth aspect is more preferred. Those aspects may be carried out alone, but two or more kinds thereof may be carried out in combination.

### 4. Method of detecting Presence or Absence of Activation of Immunoglobulin-producing Cells

In a diabetic patient, the concentration of the lambda FLC in blood is increased as compared to that in a healthy subject. Meanwhile, in the diabetic patient, the concentration of the kappa FLC in the specimen is reduced as compared to that in the healthy subject, but the sum of the concentration of the lambda FLC and the concentration of the kappa FLC in the blood is increased as compared to that in the healthy subject. It is considered from the foregoing that, in the diabetic patient, immunoglobulin-producing cells, particularly lambda chain-producing cells are activated.

The present invention relates to a method of detecting the presence or absence of activation of immunoglobulin-producing cells for a diabetic patient. The method of detecting the presence or absence of activation of immunoglobulin-producing cells for a diabetic patient includes the following five aspects.

An aspect I includes the following steps (1) to (4): (1) measuring concentrations of a kappa chain and a lambda chain, which are included in free immunoglobulin light chains, in a specimen collected from a test subject suffering from diabetes, in accordance with the "2. Method of measuring Concentrations of Kappa FLC and Lambda FLC"; (2) calculating a ratio of the concentration of the kappa chain to the concentration of the lambda chain obtained in the step (1) (kappa chain/lambda chain ratio calculated value); (3) comparing the kappa chain/lambda chain ratio calculated value obtained in the step (2) to a kappa chain/lambda chain ratio defined as a threshhold (kappa chain/lambda chain ratio threshhold value); and (4) determining that immunoglobulin-producing cells are activated in the test subject when, in the step (3), the kappa chain/lambda chain ratio calculated value is lower than the kappa chain/lambda chain ratio threshhold value.

An aspect II includes the following steps (1') to (4'): (1') measuring concentrations of a kappa chain and a lambda chain, which are included in free immunoglobulin light chains, in a specimen collected from a test subject, in accordance with the "2. Method of measuring Concentrations of Kappa FLC and Lambda FLC"; (2') calculating a ratio of the concentration of the lambda chain to the concentration of the kappa chain obtained in the step (1') (lambda chain/kappa chain ratio calculated value); (3') comparing the lambda chain/kappa chain ratio calculated value obtained in the step (2') to a lambda chain/kappa chain ratio defined as a threshhold (lambda chain/kappa chain ratio threshhold value); and (4') determining that the test subject is suffering from diabetes when, in the step (3'), the lambda chain/kappa chain ratio calculated value is higher than the lambda chain/kappa chain ratio threshhold value;

An aspect III includes the following steps (A) to (D): (A) measuring concentrations of a kappa chain and a lambda chain, which are included in free immunoglobulin light chains, in a specimen collected from a test subject, in accordance with the "2. Method of measuring Concentrations of Kappa FLC and Lambda FLC"; (B) calculating an absolute value of a difference between the concentration of the kappa chain and the concentration of the lambda chain obtained in the step (A) (kappa chain-lambda chain calculated value); (C) comparing the kappa chain-lambda chain calculated value obtained in the step (B) to an absolute value of a difference between a concentration of the kappa chain and a concentration of the lambda chain defined as a threshhold (kappa chain-lambda chain threshhold value); and (D) determining that the test subject is suffering from diabetes when, in the step (C), the kappa chain-lambda chain calculated value is lower than the kappa chain-lambda chain threshhold value.

An aspect IV includes the following steps (A') to (D'): (A') measuring concentrations of a kappa chain and a lambda chain, which are included in free immunoglobulin light chains, in a specimen collected from a test subject, in accordance with the "2. Method of measuring Concentrations of Kappa FLC and Lambda FLC"; (B') calculating an absolute value of a difference between the concentration of the lambda chain and the concentration of the kappa chain obtained in the step (A') (lambda chain-kappa chain calculated value); (C') comparing the lambda chain-kappa chain calculated value obtained in the step (B') to an absolute value of a difference between a concentration of the lambda chain and a concentration of the kappa chain defined as a threshhold (lambda chain-kappa chain threshhold value); and (D') determining that the test subject is suffering from diabetes when, in the step (C'), the lambda chain-kappa chain calculated value is lower than the lambda chain-kappa chain threshhold value.

An aspect V includes the following steps (1") to (4"): (1") measuring concentrations of a kappa chain and a lambda chain, which are included in free immunoglobulin light chains, in a specimen collected from a test subject suffering from diabetes, in accordance with the "2. Method of measuring Concentrations of Kappa FLC and Lambda FLC"; (2") calculating a sum of the concentration of the kappa chain and the concentration of the lambda chain obtained in the step (1") (kappa chain+lambda chain calculated value); (3") comparing the kappa chain+lambda chain calculated value obtained in the step (2") to a kappa chain+lambda chain value defined as a threshhold (kappa chain+lambda chain threshhold value); and (4") determining that immunoglobulin-producing cells are activated in the test subject when, in the step (3"), the kappa chain+lambda chain calculated value is higher than the kappa chain+lambda chain threshhold value.

An aspect VI includes the following steps (i) to (iii): (i) measuring a concentration of a lambda chain, which is included in free immunoglobulin light chains, in a specimen collected from a test subject suffering from diabetes, in accordance with the "2. Method of measuring Concentrations of Kappa FLC and Lambda FLC"; (ii) comparing the concentration of the lambda chain (lambda chain concentration) obtained in the step (i) to a concentration of the lambda chain defined as a threshhold (lambda chain threshhold value); and (iii) determining that immunoglobulin-producing cells are activated in the test subject when the lambda chain concentration is higher than the lambda chain threshhold value.

An aspect VII includes the following steps (I) to (III) :
(I) measuring a concentration of a kappa chain, which is included in free immunoglobulin light chains, in a specimen collected from a test subject suffering from diabetes, in accordance with the "2. Method of measuring Concentrations of Kappa FLC and Lambda FLC"; (II) comparing the concentration of the kappa chain (kappa chain concentration) obtained in the step (I) to a concentration of the kappa chain defined as a threshhold (kappa chain threshhold value); and (III) determining that immunoglobulin-producing cells are activated in the test subject when the kappa chain concentration is lower than the kappa chain threshhold value.

The aspects I to VII are based on the following fact: in the diabetic patient, the concentration of the kappa FLC in the specimen is reduced as compared to that in a healthy subject, and the concentration of the lambda FLC in blood is increased as compared to that in the healthy subject. Therefore, the aspects I to VII may each be carried out alone, but two or more kinds selected from the group consisting of the aspects I to VII may be carried out in combination. In addition, of the above-mentioned aspects, as the method of detecting the presence or absence of activation of immunoglobulin-producing cells for a diabetic patient, the aspect I, the aspect III, the aspect V, or the aspect VI is preferred. The aspect I, the aspect III, the aspect V, or the aspect VI may be carried out alone, but two or more kinds thereof may be carried out in combination.

### 5. Method of treating Diabetes

The "3. Method of determining Morbidity of Diabetes" may further include a step of treating diabetes. Specifically, when it is determined that the test subject has diabetes in the determination method, the method includes the step of treating diabetes.

The treatment of diabetes may be performed in accordance with a known method. Examples of the method for the treatment of diabetes may include diet therapy, exercise therapy, and the administration of a drug. Examples of the drug may include: insulin resistance improving drugs, such as a thiazolidine drug and a biguanide drug; insulin secretagogue drugs, such as a glinide drug, a sulfonylurea drug, and a DPP-4 inhibitor; and glucose absorption/excretion controlling drugs, such as an SGLT2 inhibitor and an α-glucosidase inhibitor.

In addition, the "4. Method of detecting Presence or Absence of Activation of Immunoglobulin-producing Cells" further includes a step of suppressing the activation of immunoglobulin-producing cells when it is determined that immunoglobulin-producing cells are activated in the test subject. The suppression of the activation of immunoglobulin-producing cells may be performed in accordance with a known method. The method is, for example, immunosuppressive therapy.

### 6. Computer Program

The present invention includes, as another aspect, a computer program for causing a computer including a processor and a memory under the control of the processor to execute: the steps (1) to (4); the steps (1') to (4'); the steps (A) to (D); or the steps (A') to (D') described in the "3. Method of determining Morbidity of Diabetes."

The present invention also includes, as another aspect, a computer program for causing a computer including a processor and a memory under the control of the processor to execute: the steps (1) to (4); the steps (1') to (4'); the steps (A) to (D); or the steps (A') to (D') described in the "4. Method of detecting Presence or Absence of Activation of Immunoglobulin-producing Cells."

Those computer programs may each be recorded on a recording medium.

### 7. Test Reagent

The present invention relates to a test reagent to be used in "3. Method of determining Morbidity of Diabetes" and "4. Method of detecting Presence or Absence of Activation of Immunoglobulin-producing Cells."

The test reagent includes the following two aspects.

A test reagent of an aspect 1 includes a free immunoglobulin kappa light chain antibody (anti-kappa chain antibody).

A test reagent of an aspect 2 includes a free immunoglobulin lambda light chain antibody (anti-lambda chain antibody).

As the free immunoglobulin kappa light chain antibody and the free immunoglobulin lambda light chain antibody, the ones described in the "1. Description of Terms" may be used.

The test reagent only needs to include at least one kind of anti-kappa chain antibody or anti-lambda chain antibody. When the anti-kappa chain antibody or the anti-lambda chain antibody is polyclonal antibodies, the polyclonal antibodies may be obtained by immunization with one kind of antigen, or the polyclonal antibodies may be obtained by concurrently immunizing the same individual with two or more kinds of antigens. In addition, respective polyclonal antibodies obtained by inoculating two or more kinds of antigens into animals different from each other may be mixed with each other. When the anti-kappa chain antibody is a monoclonal antibody, the monoclonal antibody may be produced from one kind of hybridoma, but the monoclonal antibody may be produced from two or more kinds of hybridomas so as to include two or more kinds of a plurality of monoclonal antibodies, the monoclonal antibodies recognizing epitopes identical to or different from each other. In addition, one or more kinds of polyclonal antibodies and one or more kinds of monoclonal antibodies may be included as a mixture.

The form of the anti-kappa chain antibody or anti-lambda chain antibody included in the test reagent is not particularly limited, and may be a dry state or liquid state of, for example, antiserum or ascites containing each antibody. In addition, the form of each antibody may be a purified antibody, an immunoglobulin fraction containing each antibody, or a dry state or aqueous solution of an IgG fraction containing each antibody.

When the form is the dry state or liquid state of antiserum or ascites containing each antibody, the form may further contain at least one of: stabilizers, such as β-mercaptoethanol and DTT; protective agents, such as albumin; surfactants, such as polyoxyethylene (20) sorbitan monolaurate and polyoxyethylene (10) octylphenyl ether; and preservatives, such as sodium azide. In addition, when the form of each antibody is the purified antibody, the immunoglobulin fraction containing each antibody, or the dry state or aqueous solution of the IgG fraction containing each antibody, the form may further contain at least one of: buffer components, such as a phosphate buffer; stabilizers, such as β-mercaptoethanol and DTT; protective agents, such as albumin; salts, such as sodium chloride; surfactants and the like, such as polyoxyethylene (20) sorbitan monolaurate and polyoxyethylene (10) octylphenyl ether; and preservatives, such as sodium azide.

The anti-kappa chain antibody or anti-lambda chain antibody included in the test reagent may be unlabeled, or may be labeled with biotin or the above-mentioned labeling substance, but is preferably labeled with biotin or the above-mentioned labeling substance. As the labeling substance, the one exemplified in the section "2. Method of measuring Concentrations of Kappa FLC and Lambda FLC" may be used, and alkaline phosphatase is preferred. In addition, in the present invention, each antibody may be provided in a state of being immobilized to the surface of a solid phase or the like. The solid phase and the immobilization are as exemplified in the section "2. Method of measuring Concentrations of Kappa FLC and Lambda FLC." The solid phase is preferably fluorescent beads or magnetic beads.

### 8. Test Kit

The present invention relates to a test kit including the test reagent described in the "7. Test reagent".

The test kit includes the following three aspects.

An aspect 1 is a test kit including a free immunoglobulin kappa light chain capture antibody and a free immunoglobulin kappa light chain detection antibody.

An aspect 2 is a test kit including a free immunoglobulin lambda light chain capture antibody and a free immunoglobulin lambda light chain detection antibody.

An aspect 3 is a test kit including a free immunoglobulin kappa light chain capture antibody, a free immunoglobulin kappa light chain detection antibody, a free immunoglobulin lambda light chain capture antibody, and a free immunoglobulin lambda light chain detection antibody.

More specifically, the test kit includes a test reagent including the anti-kappa chain antibody and/or anti-lambda chain antibody labeled with the labeling substance (preferably biotin) exemplified in the section "2. Method of measuring Concentrations of Kappa FLC and Lambda FLC" to be used as the capture antibody, or a test reagent including the anti-kappa chain antibody and/or anti-lambda chain antibody labeled with the labeling substance (preferably alkaline phosphatase or the fluorescent substance) exemplified in the section "2. Method of measuring Concentrations of Kappa FLC and Lambda FLC" to be used as the detection antibody. This embodiment may further include a solid phase, preferably a solid phase having bound thereto an avidin, more preferably bound beads or magnetic beads having bound thereto an avidin. In addition, the capture antibody may be provided in a state of being immobilized to a solid phase. This embodiment may further include a substrate. The substrate is preferably CDP-Star (trademark).

The details of the labeling substance, the solid phase, and the substrate are as described in "2. Method of measuring Concentrations of Kappa FLC and Lambda FLC," and the description may be incorporated herein.

When the capture antibody is bound to the solid phase in advance, the test kit according to this embodiment may include the solid phase having the capture antibody immobilized thereto, the detection antibody, and the labeling substance. The detection antibody and the labeling substance may be contained in separate containers, or may be contained in the same container. When the labeling substance is an enzyme and the test kit further includes the substrate, the enzyme and the substrate need to be contained in separate containers. When provided to a user, at least two kinds of the solid phase, the detection antibody, and the labeling substance may be packaged together, or may be separately packaged.

When the capture antibody is not bound to the solid phase in advance, the test kit according to this embodiment may include the solid phase, the capture antibody, the detection antibody, and the labeling substance. At least two kinds of the capture antibody, the detection antibody, and the labeling substance may be contained in the same container, or may be contained in separate containers. When the labeling substance is an enzyme and the test kit further includes the substrate, the enzyme and the substrate need to be contained in separate containers. When provided to a user, at least two kinds of the solid phase, the capture antibody, the detection antibody, and the labeling substance may be packaged together, or may be separately packaged.

In addition, when the test kit is a kit for performing a competitive ELISA method, the kit may include the capture antibody and the competitor described in "2. Method of measuring Concentrations of Kappa FLC and Lambda FLC." In this case, the capture antibody may be bound to a solid phase or may not be bound thereto.

### Examples

Now, the present invention is described in more detail by way of Example, but the present invention is not to be construed as being limited to the aspect of Example.

### Example: Measurement of Kappa FLC and Lambda FLC Concentrations in Serum

### 1. Method

Kappa FLC and lambda FLC concentrations in serum were measured using 95 diabetic patients and 75 healthy subjects as test subjects. The healthy subjects are ones who are not suspected of having diseases including not only diabetes but also other diseases. Immediately after collection of blood from each of the test subjects, serum was separated therefrom, and the separated serum was immediately frozen at -80°C and stored at - 80°C until the measurement. The measurement was commissioned to a group including the authors of Non-patent Literature 3 and was performed by the method described in Non-patent Literature 3.

Through the use of values for the kappa FLC and lambda FLC concentrations obtained by the measurement, for each of the diabetic patients and the healthy subjects, the ratio of the concentration of the kappa chain to the concentration of the lambda chain (kappa chain/lambda chain ratio calculated value), the absolute value of a difference between the concentration of the kappa chain and the concentration of the lambda chain (kappa chain-lambda chain calculated value), and the sum of the concentration of the kappa chain and the concentration of the lambda chain (kappa chain+lambda chain calculated value) were calculated.

For statistical analysis, StatFlex was used to perform basic statistics (parametric method) and independent multiple group comparison between two groups (two-sample t-test: Welch's method). In addition, for sensitivity and specificity tests, ROC analysis was performed to prepare ROC curves and obtain AUC values.

### 2. Results

### (1) Basic Statistics and Independent Multiple Group Comparison between Two Groups

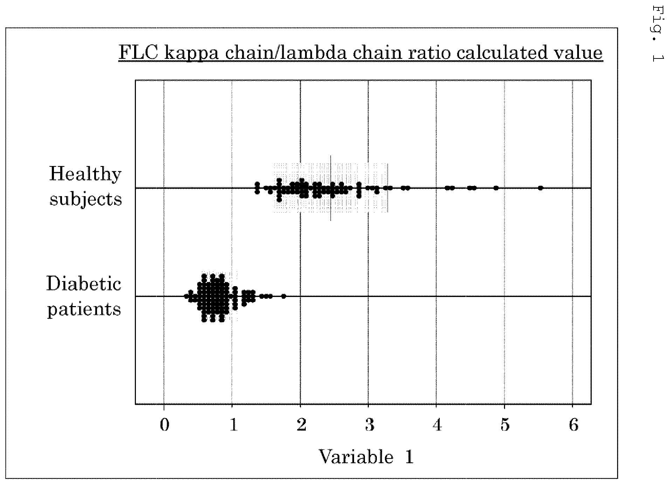

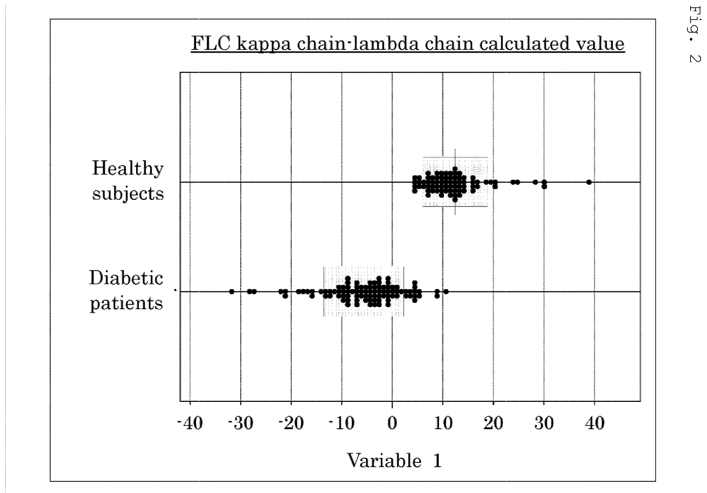

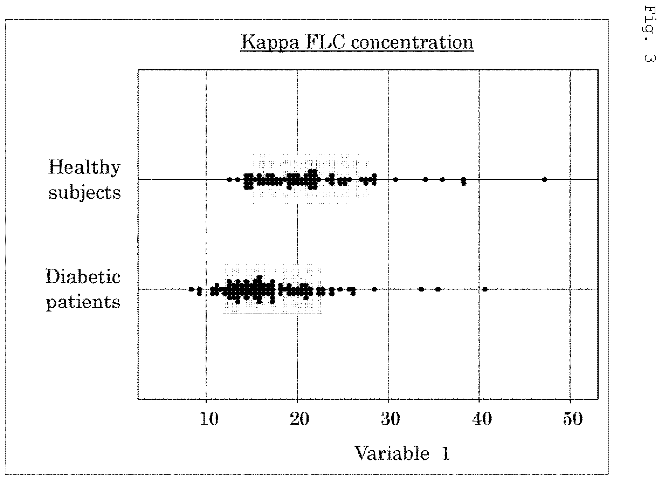

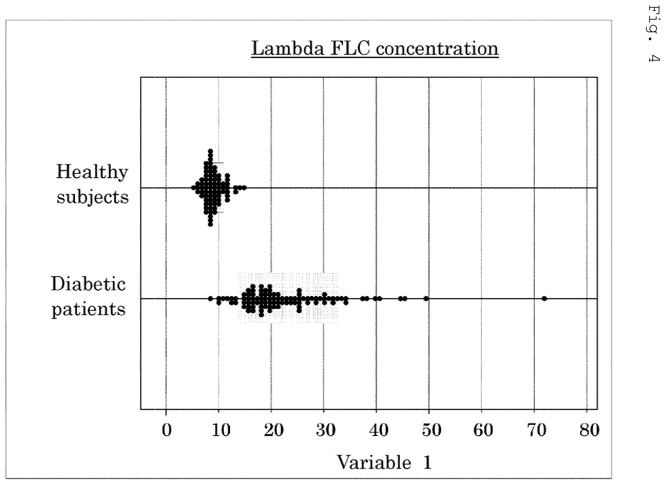

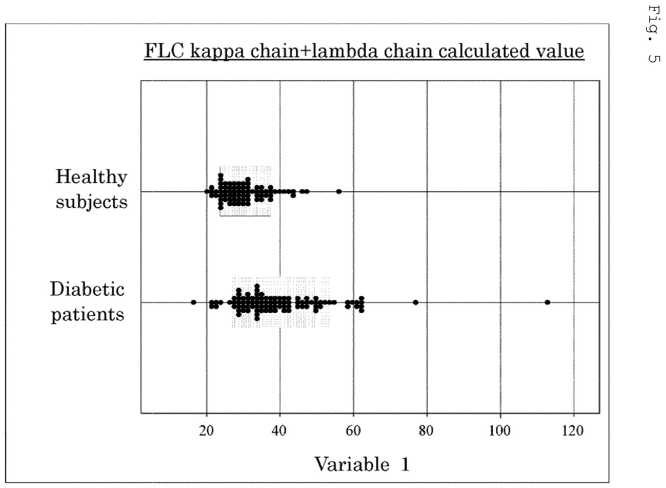

The results of the basic statistics and the independent multiple group comparison between two groups are shown in Table 2. In addition, distributions of the values in the diabetic patients and the healthy subjects are shown in FIG. 1 to FIG. 5. The kappa FLC concentration, the kappa chain/lambda chain ratio calculated value, and the kappa chain-lambda chain calculated value were significantly reduced in the diabetic patients as compared to those in the healthy subjects. In addition, the lambda FLC concentration and the kappa chain+lambda chain calculated value were significantly increased in the diabetic patients as compared to those in the healthy subjects.

**Table 2**

| | Diabetic patient | Healthy subject | p-value |
|---|---|---|---|
| Kappa chain/lambda chain ratio calculated value | 0.82±0.27 | 2.45±0.84 | <0.00001 |
| Kappa chain-lambda chain calculated value | -5.59±7.95 | 12.42±6.38 | <0.00001 |
| Kappa FLC concentration (mg/L) | 17.25±5.48 | 21.50±6.36 | 0.00001 |
| Lambda FLC concentration (mg/L) | 22.84±9.66 | 9.08±1.89 | <0.000001 |
| Kappa chain+lambda chain calculated value | 40.09±13.55 | 30.58±6.87 | <0.00001 |

### (2) ROC Analysis

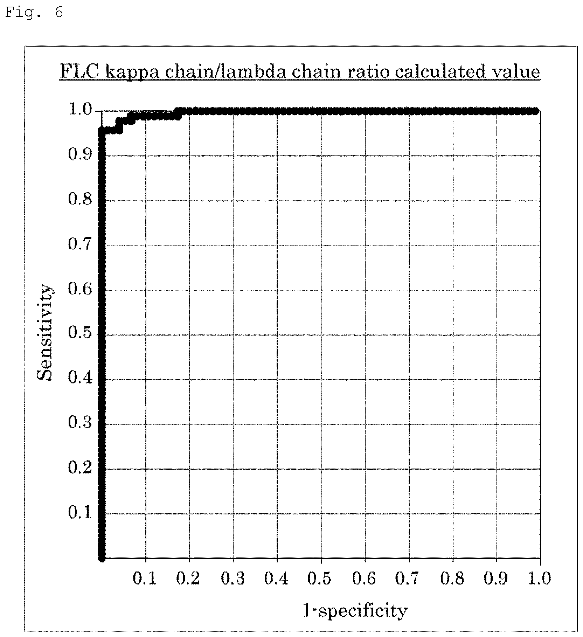

The AUC values obtained by the ROC analysis of the diabetic patient group and the healthy subject group are shown in Table 3. In addition, the ROC curves are shown in FIG. 6 to FIG. 10. The AUC values for the lambda FLC concentration, the kappa chain/lambda chain ratio calculated value, and the kappa chain-lambda chain calculated value were 0.98, 0.97, and 0.99, all of which were classified as high accuracy. The AUC values for the kappa FLC concentration and the kappa chain+lambda chain calculated value were 0.72 and 0.76, which were classified as low accuracy.

**Table 3**

| | AUC-value |
|---|---|
| Kappa chain/lambda chain ratio calculated value | 0.997 |
| Kappa chain-lambda chain calculated value | 0.99 |
| Kappa FLC concentration (mg/L) | 0.72 |
| Lambda FLC concentration (mg/L) | 0.98 |
| Kappa chain+lambda chain calculated value | 0.76 |

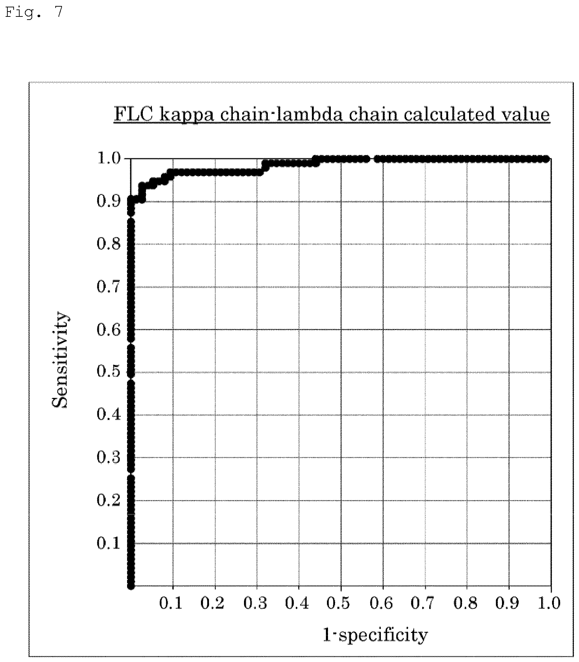

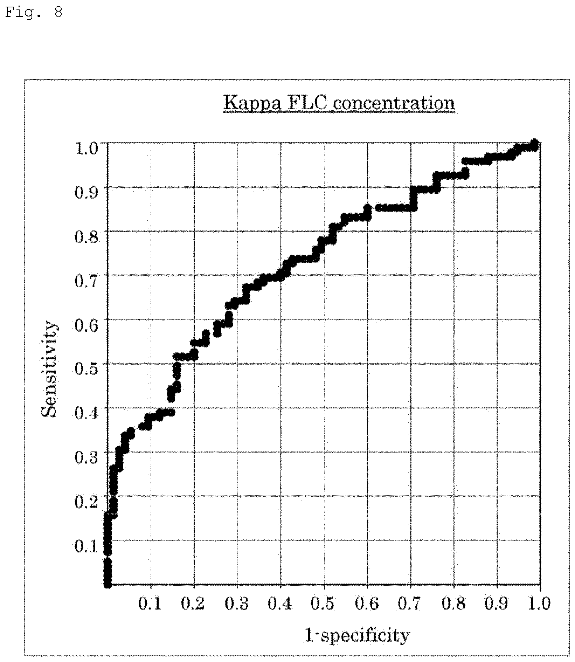

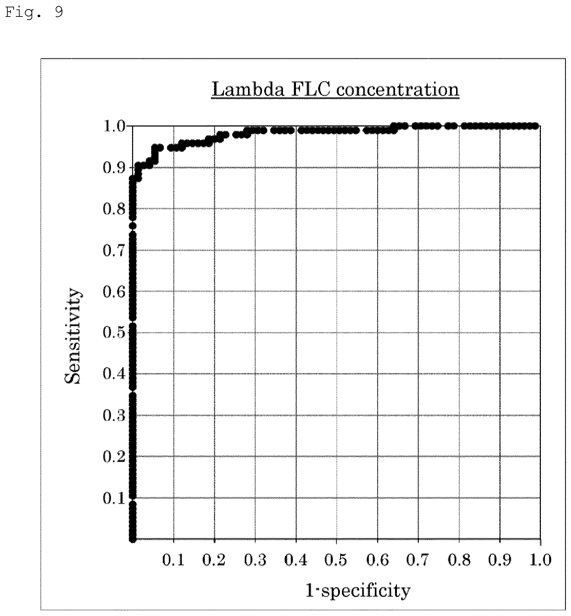

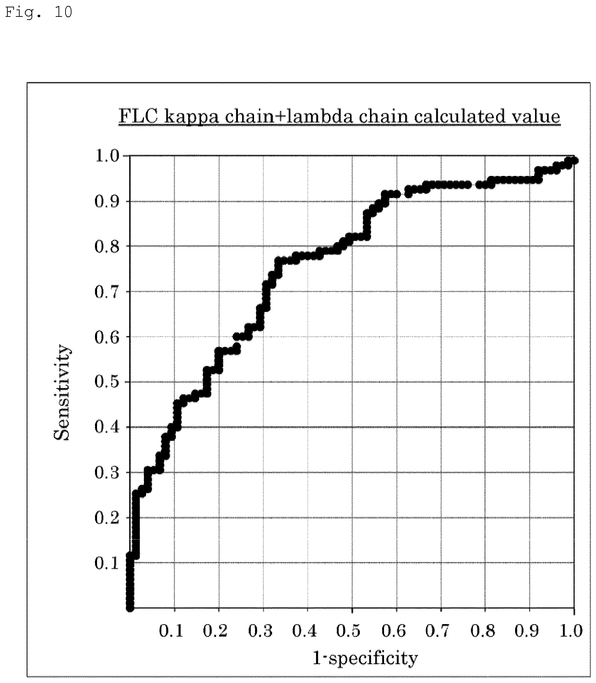

In addition, with regard to the ROC curves, as shown in FIG. 6 to FIG. 10, the graphs of the kappa chain/lambda chain ratio calculated value (FIG. 6), the kappa chain-lambda chain calculated value (FIG. 7), and the lambda FLC concentration (FIG. 9) each had a shape along the upper left corner of the graph, and hence the results of both sensitivity and specificity were extremely high in the diabetic patients. On the other hand, the ROC curves of the kappa FLC concentration (FIG. 8) and the kappa chain+lambda chain calculated value (FIG. 10) each had a shape along the diagonal line connecting the lower left and the upper right of the graph, indicating low sensitivity and specificity to the diabetic patients.

### 3. Conclusion

It was shown from the foregoing that, in the diabetic patients, the kappa FLC concentration in the blood was decreased and the lambda FLC concentration in the blood was increased. As a result, it was revealed that the kappa chain/lambda chain ratio calculated value and the kappa chain-lambda chain calculated value were decreased. In addition, as a result of the ROC analysis, it was revealed that the kappa chain/lambda chain ratio calculated value, the lambda FLC concentration, and the kappa chain-lambda chain calculated value were excellent in predicting ability and diagnostic ability for diabetes. In addition, it is apparent from those results that the lambda chain/kappa chain ratio calculated value and the lambda chain-kappa chain calculated value are also excellent in predicting ability and diagnostic ability for diabetes.

Further, the lambda FLC concentration and the kappa chain+lambda chain calculated value revealed that activation of immunoglobulin-producing cells occurred in the diabetic patients.

## Claims

1. A method of determining morbidity of diabetes for a test subject, comprising the following steps (1) to (4), steps (1') to (4'), steps (A) to (D), or steps (A') to (D'):
(1) measuring concentrations of a kappa chain and a lambda chain, which are included in free immunoglobulin light chains, in a specimen collected from a test subject;
(2) calculating a ratio of the concentration of the kappa chain to the concentration of the lambda chain obtained in the step (1) (kappa chain/lambda chain ratio calculated value);
(3) comparing the kappa chain/lambda chain ratio calculated value obtained in the step (2) to a kappa chain/lambda chain ratio defined as a threshhold (kappa chain/lambda chain ratio threshhold value); and
(4) determining that the test subject is suffering from diabetes when, in the step (3), the kappa chain/lambda chain ratio calculated value is lower than the kappa chain/lambda chain ratio threshhold value;
(1') measuring concentrations of a kappa chain and a lambda chain, which are included in free immunoglobulin light chains, in a specimen collected from a test subject;
(2') calculating a ratio of the concentration of the lambda chain to the concentration of the kappa chain obtained in the step (1') (lambda chain/kappa chain ratio calculated value);
(3') comparing the lambda chain/kappa chain ratio calculated value obtained in the step (2') to a lambda chain/kappa chain ratio defined as a threshhold (lambda chain/kappa chain ratio threshhold value); and
(4') determining that the test subject is suffering from diabetes when, in the step (3'), the lambda chain/kappa chain ratio calculated value is higher than the lambda chain/kappa chain ratio threshhold value;
(A) measuring concentrations of a kappa chain and a lambda chain, which are included in free immunoglobulin light chains, in a specimen collected from a test subject;
(B) calculating an absolute value of a difference between the concentration of the kappa chain and the concentration of the lambda chain obtained in the step (A) (kappa chain-lambda chain calculated value);
(C) comparing the kappa chain-lambda chain calculated value obtained in the step (B) to an absolute value of a difference between a concentration of the kappa chain and a concentration of the lambda chain defined as a threshhold (kappa chain-lambda chain threshhold value); and
(D) determining that the test subject is suffering from diabetes when, in the step (C), the kappa chain-lambda chain calculated value is lower than the kappa chain-lambda chain threshhold value; or
(A') measuring concentrations of a kappa chain and a lambda chain, which are included in free immunoglobulin light chains, in a specimen collected from a test subject;
(B') calculating an absolute value of a difference between the concentration of the lambda chain and the concentration of the kappa chain obtained in the step (A') (lambda chain-kappa chain calculated value);
(C') comparing the lambda chain-kappa chain calculated value obtained in the step (B') to an absolute value of a difference between a concentration of the lambda chain and a concentration of the kappa chain defined as a threshhold (lambda chain-kappa chain threshhold value); and
(D') determining that the test subject is suffering from diabetes when, in the step (C'), the lambda chain-kappa chain calculated value is lower than the lambda chain-kappa chain threshhold value.

2. A method of determining morbidity of diabetes for a test subject, comprising the following steps (i) to (iii):
(i) measuring a concentration of a lambda chain, which is included in free immunoglobulin light chains, in a specimen collected from a test subject;
(ii) comparing the concentration of the lambda chain (lambda chain concentration) obtained in the step (i) to a concentration of the lambda chain defined as a threshhold (lambda chain threshhold value); and
(iii) determining that the test subject is suffering from diabetes when the lambda chain concentration is higher than the lambda chain threshhold value.

3. A method of determining morbidity of diabetes for a test subject, comprising the following steps (I) to (III):
(I) measuring a concentration of a kappa chain, which is included in free immunoglobulin light chains, in a specimen collected from a test subject;
(II) comparing the concentration of the kappa chain (kappa chain concentration) obtained in the step (i) to a concentration of the kappa chain defined as a threshhold (kappa chain threshhold value); and
(III) determining that the test subject is suffering from diabetes when the kappa chain concentration is lower than the kappa chain threshhold value.

4. A method of detecting a presence or absence of activation of immunoglobulin-producing cells for a diabetic patient, comprising the following steps (1) to (4), steps (1') to (4'), steps (A) to (D), steps (A') to (D'), or steps (1") to (4") :
(1) measuring concentrations of a kappa chain and a lambda chain, which are included in free immunoglobulin light chains, in a specimen collected from a test subject suffering from diabetes;
(2) calculating a ratio of the concentration of the kappa chain to the concentration of the lambda chain obtained in the step (1) (kappa chain/lambda chain ratio calculated value);
(3) comparing the kappa chain/lambda chain ratio calculated value obtained in the step (2) to a kappa chain/lambda chain ratio defined as a threshhold (kappa chain/lambda chain ratio threshhold value); and
(4) determining that immunoglobulin-producing cells are activated in the test subject when, in the step (3), the kappa chain/lambda chain ratio calculated value is lower than the kappa chain/lambda chain ratio threshhold value;
(1') measuring concentrations of a kappa chain and a lambda chain, which are included in free immunoglobulin light chains, in a specimen collected from a test subject;
(2') calculating a ratio of the concentration of the lambda chain to the concentration of the kappa chain obtained in the step (1') (lambda chain/kappa chain ratio calculated value);
(3') comparing the lambda chain/kappa chain ratio calculated value obtained in the step (2') to a lambda chain/kappa chain ratio defined as a threshhold (lambda chain/kappa chain ratio threshhold value); and
(4') determining that immunoglobulin-producing cells are activated in the test subject when, in the step (3'), the lambda chain/kappa chain ratio calculated value is higher than the lambda chain/kappa chain ratio threshhold value;
(A) measuring concentrations of a kappa chain and a lambda chain, which are included in free immunoglobulin light chains, in a specimen collected from a test subject;
(B) calculating an absolute value of a difference between the concentration of the kappa chain and the concentration of the lambda chain obtained in the step (A) (kappa chain-lambda chain calculated value);
(C) comparing the kappa chain-lambda chain calculated value obtained in the step (B) to an absolute value of a difference between a concentration of the kappa chain and a concentration of the lambda chain defined as a threshhold (kappa chain-lambda chain threshhold value); and
(D) determining that immunoglobulin-producing cells are activated in the test subject when, in the step (C), the kappa chain-lambda chain calculated value is lower than the kappa chain-lambda chain threshhold value;
(A') measuring concentrations of a kappa chain and a lambda chain, which are included in free immunoglobulin light chains, in a specimen collected from a test subject;
(B') calculating an absolute value of a difference between the concentration of the lambda chain and the concentration of the kappa chain obtained in the step (A') (lambda chain-kappa chain calculated value);
(C') comparing the lambda chain-kappa chain calculated value obtained in the step (B') to an absolute value of a difference between a concentration of the lambda chain and a concentration of the kappa chain defined as a threshhold (lambda chain-kappa chain threshhold value); and
(D') determining that immunoglobulin-producing cells are activated in the test subject when, in the step (C'), the lambda chain-kappa chain calculated value is lower than the lambda chain-kappa chain threshhold value; or
(1") measuring concentrations of a kappa chain and a lambda chain, which are included in free immunoglobulin light chains, in a specimen collected from a test subject suffering from diabetes;
(2") calculating a sum of the concentration of the kappa chain and the concentration of the lambda chain obtained in the step (1") (kappa chain+lambda chain calculated value);
(3") comparing the kappa chain+lambda chain calculated value obtained in the step (2") to a kappa chain+lambda chain value defined as a threshhold (kappa chain+lambda chain threshhold value); and
(4") determining that immunoglobulin-producing cells are activated in the test subject when, in the step (3"), the kappa chain+lambda chain calculated value is higher than the kappa chain+lambda chain threshhold value.

5. A method of detecting a presence or absence of activation of immunoglobulin-producing cells for a diabetic patient, comprising the following steps (i) to (iii):
(i) measuring a concentration of a lambda chain, which is included in free immunoglobulin light chains, in a specimen collected from a test subject suffering from diabetes;
(ii) comparing the concentration of the lambda chain (lambda chain concentration) obtained in the step (i) to a concentration of the lambda chain defined as a threshhold (lambda chain threshhold value); and
(iii) determining that immunoglobulin-producing cells are activated in the test subject when the lambda chain concentration is higher than the lambda chain threshhold value.

6. A method of detecting a presence or absence of activation of immunoglobulin-producing cells for a diabetic patient, comprising the following steps (I) to (III):
(I) measuring a concentration of a kappa chain, which is included in free immunoglobulin light chains, in a specimen collected from a test subject suffering from diabetes;
(II) comparing the concentration of the kappa chain (kappa chain concentration) obtained in the step (I) to a concentration of the kappa chain defined as a threshhold (kappa chain threshhold value); and
(III) determining that immunoglobulin-producing cells are activated in the test subject when the kappa chain concentration is lower than the kappa chain threshhold value.

7. A test kit for carrying out the method of claim 1 or 4, comprising:
a free immunoglobulin kappa light chain capture antibody;
a free immunoglobulin kappa light chain detection antibody;
a free immunoglobulin lambda light chain capture antibody; and
a free immunoglobulin lambda light chain detection antibody.

8. A test kit for carrying out the method of claim 1, 2, 4, or 5, comprising:
a free immunoglobulin lambda light chain capture antibody; and
a free immunoglobulin lambda light chain detection antibody.

9. A test kit for carrying out the method of claim 1, 3, 4, or 6, comprising:
a free immunoglobulin kappa light chain capture antibody; and
a free immunoglobulin kappa light chain detection antibody.

10. A test reagent for carrying out the method of claim 1, 2, 4, or 5, comprising a free immunoglobulin lambda light chain antibody.

11. A test reagent for carrying out the method of claim 1, 3, 4, or 6, comprising a free immunoglobulin kappa light chain antibody.
